# EUROPEAN PATENT SPECIFICATION

(11) **EP 0 538 271 B1**
(45) Date of publication and mention of the grant of the patent: **21.12.1994**
(21) Application number: 91910515.5
(22) Date of filing: 17.05.1991
(51) Int. Cl.: A61M 29/00, A61M 25/01

(54) **TRACKING GUIDEWIRE**
SPURFÜHRBARER FÜHRUNGSDRAHT
BROCHE DE GUIDAGE

(30) Priority: 11.06.1990 US 535932
(43) Date of publication of application: 28.04.1993
(73) Proprietor: SCHNEIDER (USA) INC., Plymouth, Minnesota 55442 (US)
(72) Inventor: SHOCKEY, Rick, L., Eagan, MN 55123 (US)
(74) Representative: Wood, David John
(86) International application number: US9103509
(87) International publication number: WO9119528

(56) References cited:
- EP-A- 363 661
- FR-A- 2 290 917
- US-A- 3 196 876
- US-A- 3 999 551
- US-A- 4 388 076

## Description

### Technical Field

This invention relates generally to a tracking guidewire, and in particular to an occlusion-penetrable guidewire having a lumen throughout its entire length into which a stylet can be inserted, and having a distal end portion with an arcuate tip and a diameter greater than that of the immediately proximal wire as disclosed in claim 1.

### Background Art

Vessel entry for treatment of certain untoward health conditions is a common practice. Such entry can include insertion into a blood vessel of a guidewire whose distal end is expected to reach a certain site within the body and have utility thereafter as required. Many times, however, a blood vessel may be completely or almost completely occluded, thereby rendering it substantially impossible to advance a guidewire there beyond to a designated site without first employing a separate procedure to remove the occlusion.

An example of the prior art is given in US-A-3 196 876, which however discloses a dilator for exploration of the common duct comprising a hollow sleeve of malleable material and a rigid probe member made of metal, the probe serving as a guide for the sleeve

It is therefore a primary object of the present invention to provide a guidewire having a lumen running its entire length and a distal end portion capable of penetrating a vascular occlusion. Another object of the present invention is to provide such a guidewire wherein the tip of the distal end portion is arcuate and the diameter of the distal end portion is greater than that of the immediately proximal wire. Yet another object of the present invention is to provide a guidewire assembly wherein a stylet can be removably inserted into the length of the guidewire lumen to thereby enhance guidewire structure. These and other objects will become apparent throughout the description which now follows.

### Disclosure of Invention

The present invention is a guidewire for placement within a blood vessel for penetrating an occlusion in the vessel. The guidewire comprises a length of flexible wire having a concentric lumen running its entire length. At its proximal end the wire has a hub opening to the lumen, and at its distal end it has a distal end portion which has an arcuate tip and a diameter greater than the diameter of the wire immediately proximal thereto. The invention additionally includes a guidewire assembly which comprises the guidewire and a flexible stylet substantially the length of the wire for removable insertion into the lumen of the wire for substantially its entire length. Such stylet placement provides a greater stiffness and structural integrity to the guidewire.

Finally a method of using the invention for penetrating an occlusion in a blood vessel comprises inserting the guidewire or guidewire assembly into an occluded blood vessel and positioning the arcuate tip of the distal end portion against the proximal wall of the occlusion. After such positioning, a dottering action is provided to the guidewire or guidewire assembly for a sufficient period of time whereby the repeated impingement upon the occlusion results in penetration thereof. Once the distal portion of the guidewire or guidewire assembly has passed through the occlusion, and the stylet has been withdrawn in the case of the guidewire assembly, a contrast medium can be injected into the lumen to confirm guidewire positioning within the vessel.

### Brief Description of Drawings

Presently preferred embodiments of the invention are illustrated in the accompanying drawings in which:
Figure 1 is a side elevation view of a guidewire;
Figure 2 is a side elevation view partially in section of a guidewire assembly;
Figure 3 is a side elevation view of a stylet;
Figure 4 is a side elevation view of a second embodiment of a distal end of a guidewire; and
Figure 5 is a side elevation view of a third embodiment of a distal end of a guidewire.

### Modes for Carrying Out the Invention

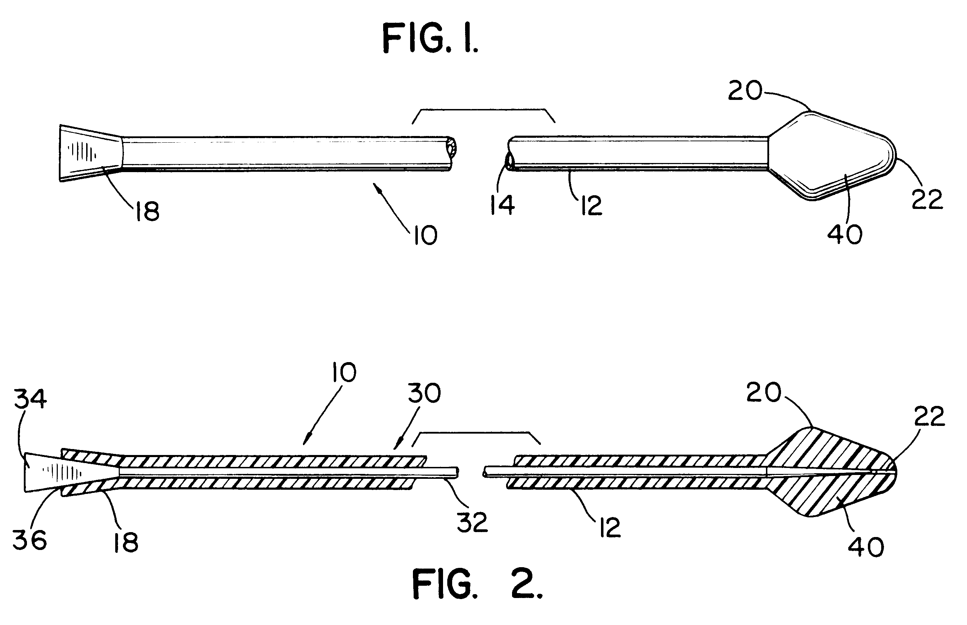

Referring to Figure 1, a guidewire 10 is illustrated. The guidewire 10 comprises a length of flexible wire 12 having a concentric lumen 14 running the entire length of the wire 12 and a proximal end hub 18 which opens to the lumen 14. The opposite end of the wire 12 has a distal end portion 20 which has an arcuate tip 22 and a diameter greater than the diameter of the wire 12 immediately proximal to the end portion 20. The guidewire 10 can be conventionally constructed of metal core, and is preferably constructed of metal coils.

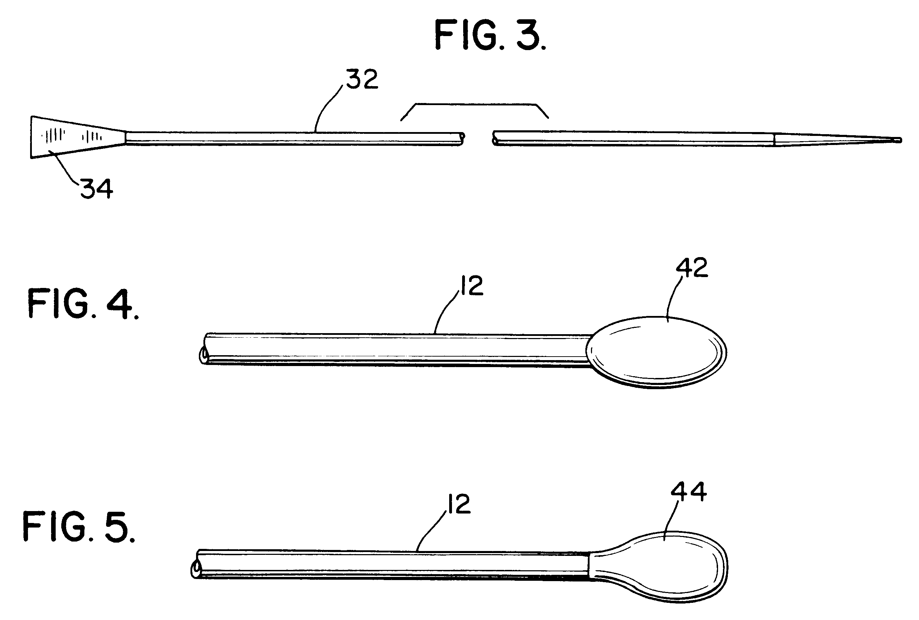

Figure 2 illustrates a guidewire assembly 30 comprising a guidewire 10, as shown in Figure 1, and a flexible stylet 32, as shown in Figure 3, disposed within the lumen 14 of the wire 12. The stylet 32 is substantially the length of the wire 12 and additionally has a proximal end member 34 whose shape is complimentary to the interior wall 36 of the hub 18. The hub 18 is provided with locking means such as conventional Luer locking threads which securely maintains the stylet 32 within the lumen 14, yet provides releasability for withdrawal of the stylet 32 as required. The stylet 32 can be constructed of metal or polymer core, and is preferably constructed of metal.

As earlier noted, the distal end portion 20 of the wire 12 has an arcuate tip 22 and has a diameter which is greater than that of the wire 12 immediately proximal thereto. Three non-limiting examples of preferred shapes of the end portion 20 are illustrated in Figures 1, 2, 4 and 5. Specifically, Figures 1 and 2 show an arrow shape 40; Figure 4 shows an elliptical shape 42; and Figure 5 illustrates a tear-drop shape 44. It is to be understood, of course, that shapes other than those illustrated can be employed as long as an arcuate tip is provided to thereby enhance physical intrusion of an occlusion. The various shapes of the respective distal end portions are attained in the manufacturing process which can include EDM machining and grinding.

In operation, the guidewire 10, and in particular its distal arcuate tip 22 and distal end portion 20, functions to penetrate an occlusion in a blood vessel. The user inserts the guidewire 10 into a blood vessel and positions the arcuate tip 22 against the proximal wall of an occlusion. Once placed, the guidewire 10 is subjected to a dottering action by the user to effectuate a repeated impinging action upon the occlusion by the distal end portion 20 for a period of time sufficient to penetrate the entire length of the occlusion and thereby permit continued travel of the guidewire 10 itself or of other apparatus. Should an occlusion exhibit a resistance too great to permit the guidewire 10 alone to penetrate, the stylet 32 is inserted into the length of the lumen 14 and locked into place. The inserted stylet 32 provides a greater stiffness and structure to the guidewire 10, thereby providing a more aggressive impingement action on the occlusion during dottering to accomplish penetration thereof. Once the distal end portion 20 of the guidewire 10 is through the stenosis, the stylet 32 can be removed from the lumen 14 and a contrast medium can injected into the lumen 14 to thereby confirm the true position of the distal end portion 20.

While an illustrative and presently preferred embodiment of the invention has been described in detail herein, it is to be understood that the inventive concepts may be otherwise variously embodied and employed within the scope of the appended claims.

## Claims

1. A guidewire assembly (10) for placement within a blood vessel for penetrating an occlusion therein, the guidewire assembly (10) comprising:
a length of flexible wire (12) having a distal end portion (20) having an arcuate tip (22) and a diameter greater than the diameter of the wire (12) immediately proximal thereto; characterized in that the wire (12) has a concentric lumen (14) running the entire length of the wire (12), with said wire (12) having a proximal end hub (18) opening to the lumen (14) and the assembly (10) comprises
a flexible stylet (32) substantially the length of the wire (12) and additionally having a proximal end member (34) complimentarily shaped to the interior wall (36) of the hub (18), said stylet (32) having a diameter less than the diameter of the lumen (14) of the wire (12) and removably insertable within the lumen (14) substantially throughout the entire length of said lumen (14).

2. A guidewire assembly (10) as claimed in claim 1 and having in addition locking means disposed at the proximal end hub (18) opening of the wire (12) for releasably securing the proximal end member (34) of the stylet (32) within at least a portion of the hub (18).

3. A guidewire assembly (10) as claimed in claim 2 wherein the distal end portion (20) of the wire (12) is in the shape of an arrow (40).

4. A guidewire assembly (10) as claimed in claim 2 wherein the distal end portion (20) of the wire (12) is in the shape of an ellipse (42).

5. A guidewire assembly (10) as claimed in claim 2 wherein the distal end portion (20) of the wire (12) is in the shape of a tear drop (44).

6. A guidewire assembly (10) as claimed in claim 1 wherein the distal end portion (20) of the wire (12) is in the shape of an arrow (40).

7. A guidewire assembly (10) as claimed in claim 1 wherein the distal end portion (20) of the wire (12) is in the shape of an ellipse (42).

8. A guidewire assembly (10) as claimed in claim 1 wherein the distal end portion (20) of the wire (12) is in the shape of a tear drop (44).

## Patentansprüche

1. Führungsdrahtbauteil (10) zur Einführung in ein Blutgefäß zur Durchdringung eines Verschlusses darin, wobei das Führungsdrahtbauteil (10) die folgenden Bestandteile umfaßt:
eine (gewisse) Länge eines flexiblen Drahts (12) mit einem distalen Endteil (20) mit einer bogenförmigen Spitze (22) und einem Durchmesser über dem Durchmesser des Drahtes (12) unmittelbar proximal dazu, dadurch gekennzeichnet, daß der Draht (12) ein konzentrisches Lumen (14), das entlang der gesamten Länge des Drahts (12) verläuft, wobei der Draht (12) einen sich zum Lumen (14) hin öffnenden proximalen Endflansch (18) aufweist, besitzt und das Bauteil (10) ein im wesentlichen die Länge des Drahtes (12) und darüber hinaus ein komplementär zur Innenwand (36) des Flansches (18) geformtes proximales Endteil (34) aufweisendes flexibles Stilett (32) mit einem unter dem Durchmesser des Lumens (14) des Drahts (12) liegenden Durchmesser, wobei das Stilett im Lumen (14) im wesentlichen entlang der gesamten Länge des Lumens (14) in entfernbarer Weise einfügbar ist, umfaßt.

2. Führungsdrahtbauteil (10) nach Anspruch 1, das darüber hinaus eine an der proximalen Endflanschöffnung (18) des Drahtes (12) angeordnete Verschlußvorrichtung zur freisetzbaren Befestigung des proximalen Endteils (34) des Stiletts (32) in mindestens einem Teil des Flansches (18) umfaßt.

3. Führungsdrahtbauteil (10) nach Anspruch 2, wobei das distale Endteil (20) des Drahtes (12) die Form eines Pfeils (40) aufweist.

4. Führungsdrahtbauteil (10) nach Anspruch 2, wobei das distale Endteil (20) des Drahtes (12) die Form einer Ellipse (42) aufweist.

5. Führungsdrahtbauteil (10) nach Anspruch 2, wobei das distale Endteil (20) des Drahtes (12) die Form eines Tränentropfens (44) aufweist.

6. Führungsdrahtbauteil (10) nach Anspruch 1, wobei das distale Endteil (20) des Drahtes (12) die Form eines Pfeils (40) aufweist.

7. Führungsdrahtbauteil (10) nach Anspruch 1, wobei das distale Endteil (20) des Drahtes (12) die Form einer Ellipse (42) aufweist.

8. Führungsdrahtbauteil (10) nach Anspruch 1, wobei das distale Endteil (20) des Drahtes (12) die Form eines Tränentropfens (44) aufweist.

## Revendications

1. Ensemble de guidage de fil (10) destiné à être mis en place à l'intérieur d'un vaisseau sanguin pour pénétrer dans une occlusion se trouvant dans ce dernier, l'ensemble (10) de guidage de fil comprenant :
un tronçon de fil souple (12) ayant une partie (20) d'extrémité distale comportant un bout courbé (22) et un diamètre supérieur au diamètre du fil (12) au voisinage immédiat de son extrémité proximale, caractérisé en ce que le fil (12) a un passage concentrique (14) se prolongeant sur la totalité de la longueur du fil (12), ledit fil (12) comportant un entonnoir d'extrémité proximale (18) s'ouvrant sur le passage (14);
un stylet souple (32) ayant sensiblement la longueur du fil (12) et comportant en plus un élément d'extrémité proximale (34) de forme complémentaire de celle de la paroi intérieure (36) de l'entonnoir (18), ledit stylet (32) ayant un diamètre inférieure au diamètre du passage (14) du fil (12) et pouvant s'introduire de manière amovible à l'intérieur du passage (14) sensiblement sur la totalité de la longueur dudit passage (14).

2. Ensemble de guidage de fil (10) selon la revendication 1, comportant en plus un moyen de blocage disposé à l'ouverture de l'entonnoir (18) de l'extrémité proximale du fil (12) pour fixer de manière amovible l'élément (34) d'extrémité proximale du stylet (32) à l'intérieur d'au moins une partie de l'entonnoir (18).

3. Ensemble de guidage de fil (10) selon la revendication 2, dans lequel la partie (20) d'extrémité distale du fil (12) est en forme de flèche (40).

4. Ensemble de guidage de fil (10) selon la revendication 2, dans lequel la partie (20) d'extrémité distale du fil (12) est en forme d'ellipse (42).

5. Ensemble de guidage de fil (10) selon la revendication 2, dans lequel la partie (20) d'extrémité distale du fil (12) est en forme de goutte d'eau (44).

6. Ensemble de guidage de fil (10) selon la revendication 1, dans lequel la partie (20) d'extrémité distale du fil (12) est en forme de flèche (40).

7. Ensemble de guidage de fil (10) selon la revendication 1, dans lequel la partie (20) d'extrémité distale du fil (12) est en forme d'ellipse (42).

8. Ensemble de guidage de fil (10) selon la revendication 1, dans lequel la partie (20) d'extrémité distale du fil (12) est en forme de goutte d'eau (44).
